Europäisches Patentamt

European Patent Office

Office européen des brevets

(11) Publication number: **0 375 778 B1**

# EUROPEAN PATENT SPECIFICATION

(45) Date of publication of patent specification: **08.09.93**　(51) Int. Cl.⁵: **A61M 5/315**

(21) Application number: **88905434.2**

(22) Date of filing: **17.06.88**

(86) International application number:
**PCT/JP88/00593**

(87) International publication number:
**WO 88/10130 (29.12.88 88/28)**

(54) **MEDICAL INSTRUMENT AND PRODUCTION THEREOF.**

(30) Priority: **19.06.87 JP 152724/87**

(43) Date of publication of application:
**04.07.90 Bulletin 90/27**

(45) Publication of the grant of the patent:
**08.09.93 Bulletin 93/36**

(84) Designated Contracting States:
**BE DE FR GB IT SE**

(56) References cited:
**EP-A- 0 264 227**　　**EP-A- 0 264 273**
**JP-A-61 166 841**　　**JP-U- 5 570 151**
**JP-U-57 176 244**　　**US-A- 4 374 717**

(73) Proprietor: **TERUMO KABUSHIKI KAISHA**
**No. 44-1, Hatagaya 2-chome, Shibuya-ku**
**Tokyo 151(JP)**

(72) Inventor: **KASAI, Masaaki Terumo Kabushiki**
**Kaisha**
**1727-1, Tsuijiarai Showa-cho**
**Nakakoma-gun Yamanashi-ken 409-38(JP)**

(74) Representative: **Gillard, Marie-Louise et al**
**Cabinet Beau de Loménie 158, rue de**
**l'Université**
**F-75340 Paris Cédex 07 (FR)**

Rank Xerox (UK) Business Services
(3.10/3.6/3.3.1)

**Description**

The present invention relates to a medical instrument, and in particular to a disposable syringe which can be assembled without using a lubricant, and a method of manufacturing the same.

A disposable syringe comprises a barrel and a plunger which can be slidably inserted in the barrel. A gasket is mounted at the distal portion of the plunger so as to assure air-tightness between the barrel and the plunger.

Typical examples of a material of the barrel and the plunger are polypropylene and poly(4-methylpentene-1). The gasket is prepared from natural rubber (NR), butyl rubber (IIR), styrene-butadiene rubber (SBR), acrylonitrile-butadiene rubber (NBR), or the like by means of a vulcanizing press-molding. As an alternative method of preparing a gasket, a thermoplastic elastomer such as a styrene-butadiene-styrene block copolymer (SBS), a styrene-ethylene-butylene-styrene block copolymer (SEBS), or an ethylene-propylene copolymer (EP) may be injection-molded.

When the barrel made of the above material is combined with the gasket made of the above material, a considerably high friction resistance is generated at a contact portion between the barrel and the gasket. In this state, the plunger cannot be slid inside the barrel. In the worst case, it is very difficult to assemble a disposable syringe by inserting the plunger into the barrel.

In conventional syringe assembly, a lubricant such as silicone oil is applied to the contact portion between the barrel and the gasket. In this case, however, the lubricant may be leaked out and mixed with a liquid in the syringe even though the amount thereof may be very small. Accordingly, the use of the lubricant is undesirable since the syringe is used to inject a drug into a human body. For this reason, a demand has arisen for a disposable syringe which allows a plunger to easily slide without using a lubricant. EP-A-0 264 273, which document forms part of the prior art according to Articles 54(2), discloses a syringe assembly which corresponds to the definitions of the preamble of claim 1

It is a first object of the present invention in consideration of the above situation to provide a medical instrument, in particular a disposable syringe which allows slidable movement of a plunger without using a lubricant.

It is a second object of the present invention to provide a gasket suitable for achieving the first object, and a method of manufacturing the same.

Disclosure of Invention

In order to achieve the first object of the present invention, it is suitable to use a lubricious material which cannot be mixed with a liquid in a syringe. A lubricating treatment free from elution of the lubricant on the surface of a gasket is performed according to the present invention.

A medical instrument according to the present invention comprises a cylindrical member and a sliding member which is adopted to slide along the inner wall of the cylindrical member, wherein the sliding member comprises a thermoplastic elastomer layer and a fluoroplastic resin layer of low dynamic friction coefficient covering a portion of the elastomer which contacts with the inner wall of the cylindrical member, said fluoroplastic resin layer having a thickness of 5 $\mu$m or less formed through low-temperature plasma polymerization.

A disposable syringe according to the present invention comprises : a synthetic resin barrel, one end of which has a nozzle and the other end of which is open ; a synthetic resin plunger which is inserted into the barrel from the open end thereof and is slidable along an inner wall surface of the barrel ; and a gasket mounted at a front end portion of the plunger so as to assure air-tightness between the plunger and the inner wall surface of the barrel, wherein an area of a surface of the gasket which is brought into contact with the inner wall surface of the barrel is covered with a film. The thickness of the lubricating film formed on the gasket surface falls within the range of 30 $\mu$m or less, preferabley 10 $\mu$m or less, and more preferably 5 $\mu$m or less. A preferable material of the lubricating film is a fluoroplastic material.

The gasket according to the present invention is mounted in contact with the inner wall surface of the barrel of the syringe and can be slid along the inner wall surface. The gasket comprises a gasket body made of an elastic material and having a predetermined shape, and a lubricating film formed on a gasket surface portion which is in slidable contact with the inner wall surface of the barrel.

A method of manufacturing the gasket mounted in contact with the inner wall surface of the barrel of the syringe so as to slide along the inner wall surface according to the present invention is characterized in that a gasket body made of an elastic material and having a predetermined shape is placed in a monomer gas atmosphere, and a plasma is generated in the atmosphere to perform low-temperature plasma polymerization of the monomer, thereby forming a lubricating film having a thickness of 5 $\mu$m or less on a surface of the gasket body.

It is known that a fluoroplastic material has low adherence and a small friction coefficient as compared with other resin materials and is used in various types of sliding surfaces and bearings. Of all fluoroplastic materials, polytetrafluoroethylene (PTFE) and a tetrafluoroethylene-hexafluoropropylene copolymer (FEP) are known as resin materials having a small dynamic friction coefficient.

Since a gasket coated with a resin having excellent lubricating properties is used in the syringe according to the present invention, the plunger can be easily inserted into the barrel during syringe assembly. In addition, during an application of the syringe, the plunger can be freely slid in the barrel without problems.

The fluoroplastic material is not dissolved in the liquid in the syringe, thus assuring safety in injection of a drug into a human body.

The fluoroplastic thin film formed on the gasket body by low-temperature plasma polymerization has high adhesion strength and cannot be easily peeled from the gasket.

In addition, the thickness of the lubricating film formed on the surface of the gasket body according to low-temperature plasma polymerization can be easily controlled.

Brief Discription of Drawings

Fig. 1 is a partial sectional view of a disposable syringe according to an embodiment of the present invention;

Fig. 2 is a partial sectional view of a gasket used in the disposable syringe shown in Fig. 1;

Fig. 3 is a partial sectional view of a gasket according to another embodiment of the present invention;

Figs. 4 and 5 are sectional views showing a method of preparing a gasket of the present invention by using a two-color injection molding method;

Fig. 6 is a schematic view showing a plasma polymerization apparatus used in the manufacture of a gasket of the present invention; and

Figs. 7A, 7B and 7C are sectional view illustrating a method of manufacturing a gasket by using another method according to the present invention.

Best Mode for Carrying out the Invention

The present invention will be described in detail with reference to preferred embodiments.

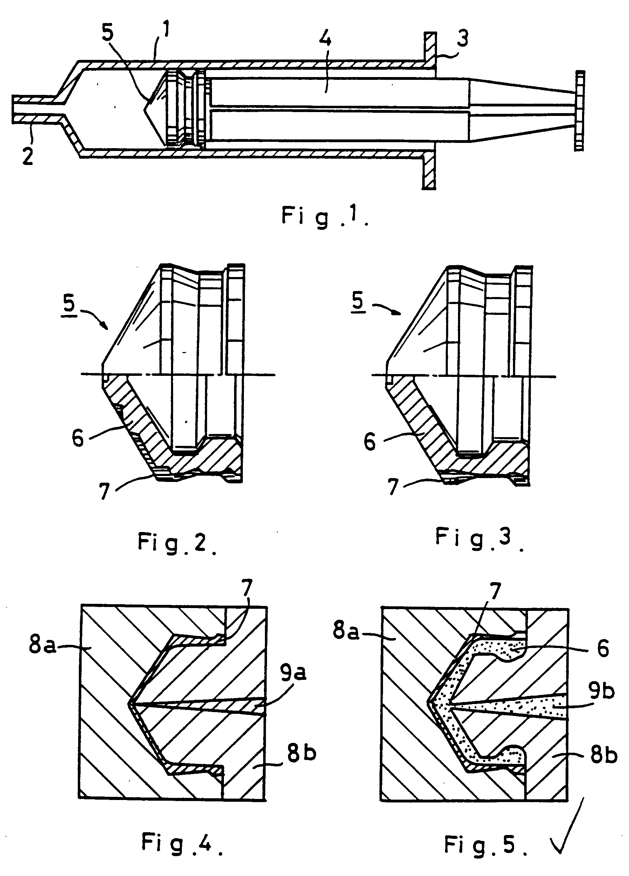

Fig. 1 is a partial sectional view of a disposable syringe according to an embodiment of the present invention. Referring to Fig. 1, reference numeral 1 denotes a synthetic resin barrel. Nozzle 2 is formed at the front end of barrel 1. A needle cannula is fitted on the nozzle when the syringe is used. The rear end of barrel 1 is open, and finger flange 3 is formed at the edge of the open end. Synthetic resin plunger 4 with a thumb rest is inserted inside barrel 1 from its rear end opening. Gasket 5 is mounted on the front end of plunger 4. Gasket 5 is brought into tight contact with the inner wall surface of barrel 1, thereby assuring air-tightness. A lubricating film (not shown) is formed on the surface of gasket 5. This lubricating film provides excellent sliding properties to gasket 5 slid along the inner wall surface of barrel 1. The details of the respective parts will be described below.

A material for barrel 1 and plunger 4 is preferably transparent and has good moldability and high impact strength. Examples of the preferable synthetic resin are polypropylene, poly(4-methylpentene-1), high-impact strength polystyrene, and polycarbonate. A more preferable material is polypropylene or poly(4-methylpentene-1).

Fig. 2 is an enlarged view showing a partial section of gasket 5. Referring to Fig. 2, reference numeral 6 denotes a gasket body. As shown in Fig. 2, the gasket body has a mushroom-like shape and has a recess for receiving the front end of plunger 4. Lubricating thin film 7 of a fluoroplastic material is formed on the surface of the gasket body. Fluoroplastic lubricating thin film 7 need not be formed on the entire surface of gasket body 6. As shown in Fig. 3, the film may be formed on gasket portions which are brought into sliding contact with the inner wall surface of barrel 1.

Gasket body 6 can be formed by injection-molding a thermoplastic elastomer. Alternatively, gasket body 6 may be formed by vulcanizing press-molding a raw rubber material. The thermoplastic elastomer may be one of the SBS, SEBS, EP, polyester, and polyurethane elastomers. At least one of the SEBS and EP elastomers is preferably used. At least one of butyl rubber, SBR, EPR, NBR, natural rubber, isoprene rubber, chloroprene rubber, and fluoro rubber is used as a cured rubber material. Preferably, at least one of SBR, NBR, natural rubber, and isoprene rubber is used.

A compression set (JIS K-6301) of gasket body 6 (at 70°C for 22 hours) is given to be 60% or less and preferably 50% or less. If the compression set exceeds 60%, the gasket is distorted such that the diameter of the gasket becomes equal to the inner diameter of the barrel. When this gasket is slid in the barrel, the peak portion of the gasket is separated from the inner wall surface of the barrel, and air-tightness cannot be assured.

A hardness value (JIS K-6301) of rubber used as gasket body 6 preferably falls within the range of 35° to 85° in JIS-A. If the hardness value exceeds 85°, it is difficult to assemble the syringe. However, if the hardness value is less than 35°, the gasket is deformed during sliding movement and it is difficult to slide the gasket body. The rubber hardness value more preferably falls within the range of 40° to 70°. At least one of SBS, SEBS, and EP copolymers having the compression set and rubber hardness values which fall within the above ranges is preferably used as a thermoplastic elastomer for a material of gasket body 6.

If the thickness of lubricating thin film 7 is excessively large, the hardness of the coated portion is too high, and air-tightness in the syringe cannot be maintained. Therefore, the thickness of lubricating thin film 7 falls within the range of 30 $\mu$m or less, preferably 10 $\mu$m or less, and more preferably 5 $\mu$m or less.

Lubricating thin film 7 preferably has a friction coefficient of 0.5 or less when it is slid along steel. If this friction coefficient exceeds 0.5, a sliding resistance value of plunger 4 is excessively increased, and the syringe cannot properly function. The sliding resistance value is defined as follows. When a plunger in an empty syringe without a needle cannula is pushed into the barrel at a predetermined speed from a maximum scale marking position to a marking position representing 10% of the maximum volume of the syringe, an initial resistance value is subtracted from the resultant resistance value. This test is repeated and differences are then averaged to obtain the "sliding resistance value".

Adhesion strength of lubricating thin film 7 on the surface of gasket body 6 falls within the range of 80/100 to 100/100 in a checkerboard test (JIS D-0202) and preferably 95/100 to 100/100. If the adhesion strength is less than 80/100, the following defect occurs. When plunger 4 is slid in the barrel, lubricating thin film 7 may be peeled from gasket body 6 since the adhesion strength between lubricating thin film 7 and gasket body 6 is weak.

Examples of the material for the lubricating thin film upon consideration of the above factors are polytetrafluoroethylene (PTFE), polyvinylidene fluoride (PVDF), polytetrafluoroethylene containing a perchloroalkoxy side chain (PFA), polychloro-trifluoroethylene, a tetrafluoroethylene-hexafluoropropylene copolyme (FEP), a polyvinylethylene fluoride-ethylene tetrafluoride copolymer, high-density polyethylene (HDPE), low-density polyethylene having a very high molecular weight, and a material containing at least one thereof.

According to the disposable syringe having the structure described above, when the plunger is slid in the barrel, an slide resistance value which allows normal use of the syringe can be obtained so that the syringe can be suitably used. It should be noted that the slide resistance value which allows normal use of the syringe is 1.8 kgf or less at a pushing speed of 200 mm/sec, although it depends on the syringe size.

A method of manufacturing gasket 5 will be described below. The following various methods can be used to manufacture gasket 5.

(A) A two-color injection molder is used to form lubricating thin film 7 by using resin 9a (e.g., polyvinylidene fluoride or high-density polyethylene) having good injection molding properties and a low dynamic friction coefficient, as shown in Fig. 4. Gasket body 6 is formed by using thermoplastic elastomer resin 9b, as shown in Fig. 5. In this case, in order to improve adhesion strength between gasket body 6 and lubricating thin film 7, a resin having a low dynamic friction coefficient may be added as thermoplastic elastomer resin 9b. Alternatively, an adhesive may be applied to the inner surface of lubricating thin film 7 prior to molding of a second color. Reference numerals 8a and 8b in Figs. 4 and 5 denote molds, respectively.

(B) A powder of a resin (e.g., fluoroplastic or polyethylene having a very high molecular weight) having a low dynamic friction coefficient providing poor fluidity is applied to molds, and subsequently a thermoplastic elastomer is injection-molded. In this case, in the same manner as in method (A), adhesion strength between gasket body 6 and lubricating thin film 7 can be improved.

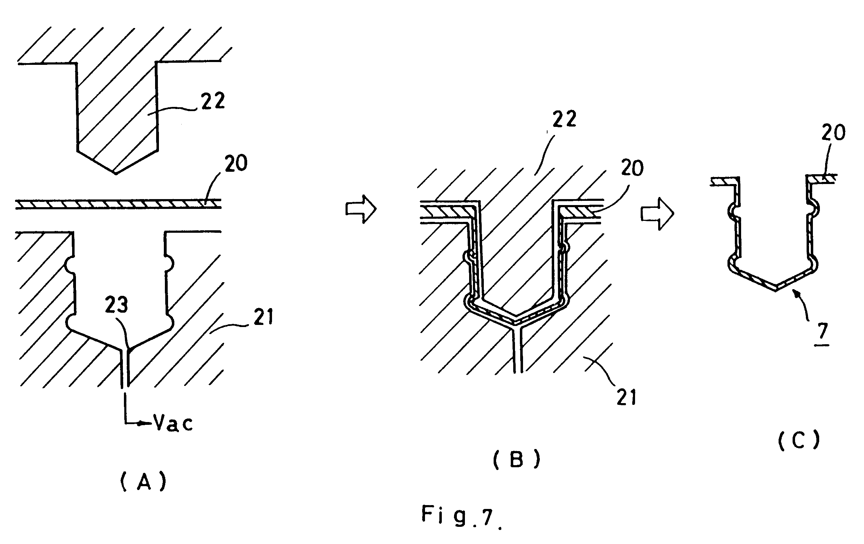

(C) As shown in Figs. 7A, 7B and 7C, a resin having a low friction coefficient is formed into a film, and the film is vacuum- or compression-molded in the molds. A thermoplastic elastomer is then injection-molded on the molded film. In the same manner as in method (A), adhesion strength between gasket body 6 and lubricating thin film 7 can be improved.

(D) Gasket body 6 is molded, and lubricating thin film 7 is formed on the surface of body 6 by powder coating, dispersion coating, or solution coating.

(E) A layer of a resin having a low dynamic friction coefficient is laminated on a thermoplastic elastomer sheet by using an adhesive, and the resultant laminated body is molded by compression molding or the like.

A most preferable method of manufacturing the gasket is the following method using low-temperature plasma polymerization. According to this method, lubricating thin film 7 is formed on the surface of gasket body 6 by using low-temperature plasma polymerization More specifically, gasket body 6 is placed in a monomer gas atmosphere for forming a lubricating thin film by polymerization, and a low-temperature plasma is generated upon application of an RF voltage. Active seeds are produced from the monomer by the behavior of the low-temperature plasma. These active seeds are reacted with each other to perform polymerization, thereby forming a lubricating polymer. This polymer is attached to and deposited on the surface of gasket body 6, and therefore lubricating thin film 7 is formed. The most preferable monomer gas in this method is a fluorine-containing monomer gas such as tetrafluoromethane, hexafluoroethylene, trifluoromethane, or pentafluoroethane.

The effects of the present invention will be described in detail by way of its examples and comparative examples.

Examples 1 - 22

Lubricating thin films 7 of a fluoroplastic material were formed on surfaces of cured rubber gasket bodies 6 by plasma polymerization, respectively (Examples 1 to 11). Each cured rubber gasket body 6 was prepared such that 30 parts by weight of carbon black HAF, 1.5 parts by weight of sulfur, 3.0 parts by weight of zinc, 0.5 parts by weight of stearic acid, and proper amounts of a curing accelerator, a curing assistant agent, and an antioxidant were added to styrene-butadiene rubber SOLPRENE 1204 (tradename) available from Nippon Elastomer K.K., and the resultant mixture was subjected to a vulcanizing press-molding at 140°C for 30 minutes. The resultant molded body was punched to prepare each cured rubber gasket body 6. In addition, 35 parts by weight of carbon black HAF, 2.3 parts by weight of sulfur, 5 parts by weight of zinc white, 2 parts by weight of stearic acid, and the proper amounts of a curing accelerator, a curing assistant agent, and an antioxidant were added to isoprene rubber JSR-IR-2200 (tradename) available from Nippon Gosei Gomu K.K., and the resultant mixture was cured and press-molded at 135°C for 30 minutes. The resultant molded body was punched to prepare a gasket body.

Gasket bodies 6 (Examples 12 to 22) were prepared by thermoplastic elastomers, following the same procedures as in Examples 1 to 11. These thermoplastic elastomer gasket bodies were prepared by injection-molding an SEBS thermoplastic elastomer AR-804 (tradename) available from Aron Kasei K.K., and an EP thermoplastic elastomer Milastomer 5510B (tradename) available from Mitsui Petrochemical Industries, Ltd.

Each gasket body 6 was placed in a reaction chamber, and the chamber was evacuated by a vacuum pump to a vacuum of 1 Torr or less. In this case, the reaction chamber may be of a flow, bell jar, or any other type. After evacuation was satisfactorily performed, fluorine-containing molecules were introduced in the chamber in the form of a gas, and the reaction chamber was kept in a vacuum of $10^{-3}$ to 10 Torr and preferably $10^{-2}$ to 5 Torr. The fluorine-containing molecules are selected from at least one of tetrafluoromethane, trifluoromethane, monochlorotrifluoromethane, hexafluoroethane, pentafluoroethane, monochloropentafluoroethane, and cycloheptafluorobutane. Preferably, at least one of tetrafluoromethane, trifluoromethane, hexafluoromethane, pentafluoroethane, and cycloheptafluorobutane is used. A plasma was generated in the reaction chamber upon application of an RF voltage while the chamber was kept at a predetermined pressure. Immediately after initiation of a reaction, polymerization occurred to form a thin film. However, a period of time required for forming a thin film having required lubricating properties varies depending on a vacuum pressure, a voltage, a monomer seed, and a monomer flow rate. Therefore, no specific reaction conditions are defined. Conditions employed in Examples 1 to 22 are summarized in Tables 1 and 2. Upon completion of the reaction, each gasket was removed after the internal pressure of the reaction chamber was returned to the atmospheric pressure.

A power source for applying the RF voltage was a combination of an RF power source SKN-05-P (tradename) and a matching box MB-500 (tradename) available from Nihon Koshuha K.K., and copper wires and copper mesh were used. Freon 14, Freon 116, and Freon 23 (tradename) available from Mitsui Fluorochemical K.K. were used as tetrafluoromethane, hexafluoroethylene, and trifluoromethane, all of which were used as monomers of fluorine-containing molecules, respectively.

Disposable syringes (Fig. 1) were assembled using the gaskets prepared in Examples 1 to 22. Polypropylene MG-3D (tradename) available from Mitsubishi Petrochemical Co., Ltd. and poly(4-methylpentene-1) RT-18 (tradename) available from Mitsui Petrochemical Industries, Ltd. were injection-molded to prepare barrels 1 and plungers 4. Surface-treated plungers 4 were engaged with corresponding gaskets 5 and were inserted in corresponding barrels 1, thereby preparing syringes. Sliding resistance

EP 0 375 778 B1

values were measured when plungers 4 were slid along the inner wall surfaces of corresponding barrels. Measurement of sliding resistance values was performed by using a compression jig Strograph-T (tradename) available from Toyo Seiki K.K. at a pushing speed of 200 mm/sec. Results are shown in Tables 1 and 2. Table 1 shows the results of cured rubber gaskets, while Table 2 shows the results of thermoplastic elastomer gaskets.

As comparative examples 1 to 8, syringes were assembled using gaskets without plasma polymerization, following the same procedures as in Examples 1 to 22. The sliding resistance values were very large, and plungers could not be fitted in the barrels.

The volume of each disposable syringe used in each example described above was 5 mℓ, but is not limited to this volume.

6

Table 1

| | Type of Monomer | Vacuum Pressure (Torr) | Applied Voltage (V) | Time (min.) | Gasket Material | Barrel Material | Sliding Resistance (kgf) | Remarks |
|---|---|---|---|---|---|---|---|---|
| Example 1 | CF$_4$ | 0.5 | 100 | 20 | SBR | PP | 0.30 | |
| "    2 | " | " | " | " | " | TPX | 0.29 | |
| "    3 | " | " | " | " | IR | PP | 0.30 | |
| "    4 | " | 1 | 50 | " | SBR | " | 0.30 | |
| "    5 | " | " | " | 30 | " | " | 0.26 | |
| "    6 | " | " | 100 | 20 | " | " | 0.19 | |
| "    7 | " | 2 | " | " | " | " | 0.16 | |
| "    8 | CF$_3$·CF$_3$ | 0.5 | " | " | " | " | 0.33 | |
| "    9 | " | " | " | 10 | " | " | 0.38 | |
| "    10 | CHF$_3$ | " | " | 20 | " | " | 0.50 | |
| "    11 | " | " | " | 30 | " | " | 0.43 | |

(Continued)

EP 0 375 778 B1

EP 0 375 778 B1

| | Type of Monomer | Vacuum Pressure (Torr) | Applied Voltage (V) | Time (min.) | Gasket Material | Barrel Material | Sliding Resistance (kgf) | Remarks |
|---|---|---|---|---|---|---|---|---|
| Comparative Example 1 | − | − | − | − | SBR | PP | Assembly failed | No plasma treatment |
| " 2 | − | − | − | − | " | TPX | " | " |
| " 3 | − | − | − | − | IR | PP | " | " |
| " 4 | $CF_4$ | 15 | 150 | 60 | SBR | " | " | No plasma generation |

Table 2

| | Type of Monomer | Vacuum Pressure (Torr) | Applied Voltage (V) | Time (min.) | Gasket Material | Barrel Material | Sliding Resistance (kgf) | Remarks |
|---|---|---|---|---|---|---|---|---|
| Example 12 | $CF_4$ | 0.1 | 100 | 20 | SEBS | PP | 0.37 | |
| " 13 | " | 0.5 | " | " | " | " | 0.27 | |
| " 14 | " | " | " | 30 | " | " | 0.23 | |
| " 15 | " | 1 | " | 20 | " | " | 0.16 | |
| " 16 | " | 0.5 | " | " | " | TPX | 0.15 | |
| " 17 | " | 1 | " | " | EP | PP | 1.29 | |
| " 18 | " | 0.5 | " | " | " | " | 0.19 | |
| " 19 | $CF_3 \cdot CF_3$ | " | " | " | SEBS | " | 0.29 | |
| " 20 | " | " | " | 10 | " | " | 0.34 | |
| " 21 | $CHF_3$ | " | " | 20 | " | " | 0.48 | |
| " 22 | " | " | " | 30 | " | " | 0.40 | |

(Continued)

EP 0 375 778 B1

| Comparative Example | Type of Monomer | Vacuum Pressure (Torr) | Applied Voltage (V) | Time (min.) | Gasket Material | Barrel Material | Sliding Resistance (kgf) | Remarks |
|---|---|---|---|---|---|---|---|---|
| 5 | – | – | – | – | SEBS | PP | Assembly failed | No plasma treatment |
| 6 | – | – | – | – | EP | " | " | " |
| 7 | – | – | – | – | SEBS | TPX | " | " |
| 8 | CF₄ | 15 | 150 | 60 | " | PP | " | No plasma generation |

Example 23

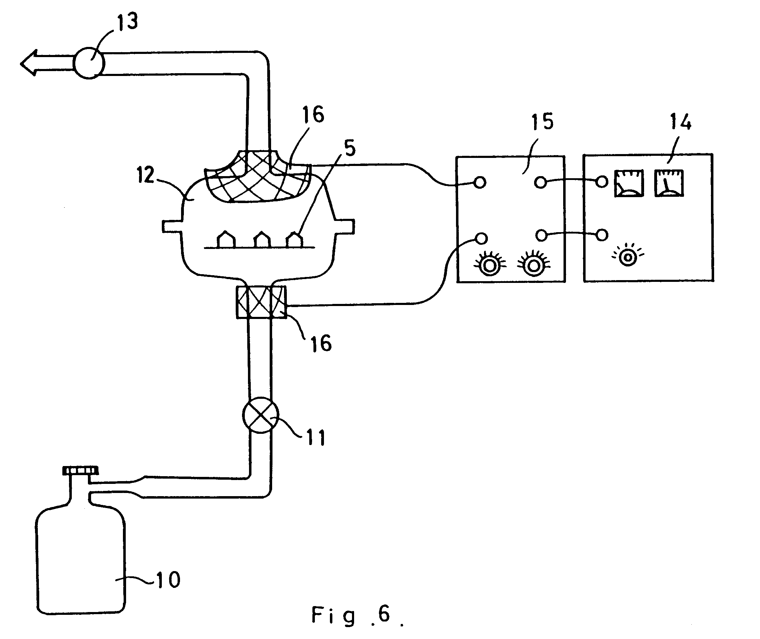

Gasket body 6 was prepared by injection-molding SEBS thermoelastic elastomer AR-804 (tradename) available from Aron Kasei K.K., and a fluoroplastic material was coated on the surface of gasket body 6 by

10

plasma polymerization. Coating of the fluoroplastic material was performed using the plasma polymerization apparatus in Fig. 6 as follows.

A monomer gas stored in monomer gas tank 10 was supplied to reaction chamber 12 through flow control valve 11. Reaction chamber 12 was evacuated by a vacuum pump through vacuum gauge 13. RF power source 14 and matching box 15 were connected to reaction chamber 12 through copper mesh 16. In Example 23, an SKN-05P (tradename) available from Nihon Koshuha K.K. and an MB-500 available from Nihon Koshuha K.K. were used as RF power source 14 and matching box 15, respectively. Tetrofluoromethane Freon 14 (tradename) available from Fluorochemical K.K. was used as a monomer gas. When plasma polymerization was performed, the reaction chamber was set in a vacuum of 1 Torr and a voltage of 100 V was applied thereto for 20 minutes.

The gasket surface-treated by the above plasma polymerization was cut, and the thickness of a film formed thereon was measured to be 0.3 to 0.5 $\mu$m by a scanning electron microscope JSM-840 (tradename) available from Nihon Denshi K.K.

A plate made of the same thermoplastic elastomer as that of the gasket was plasma-polymerized and a dynamic friction coefficient (with respect to steel) of a film formed on the plate was measured to be 0.4. A checkerboard test was conducted using a plate as in the plate obtained above, and a measurement value was 100/100.

A disposable syringe was assembled using the above gasket and barrel 1 and plunger 4. In this case, barrel 1 and plunger 4 were prepared by injection-molding MG-3D (tradename) available from Mitsubishi Petrochemical Co., Ltd. A peak diameter of the gasket was 13.2 mm and an inner diameter of the barrel was 13.0 mm. As a result, the disposable syringe could be easily assembled without using a lubricant.

The resultant syringe was sterilized with gamma rays of 2.0 Mrad using Co (cobalt) having a molecular weight of 60 as a ray source. A 22-G cannula was attached to the nozzle of the syringe, and 5 m$\ell$ of phys1ological saline Terumo Seishoku (tradename) available from Terumo K.K. was drawn into the syringe and injected in a vein of a dog. The syringe could be properly used without problems.

Example 24

A surface-treated gasket was prepared following the same procedures as in Example 23 except that hexafluoroethane Freon 116 (tradename) available from Mitsui Fluorochemical K.K. was used as a monomer for plasma polymerization. The same results as in the measurement of the film thickness and the checkerboard test in Example 23 were obtained. A dynamic friction coefficient (with respect to steel) was 0.5. No problems occurred in assembly of a disposable syringe, gamma-ray sterilization, and venous injection to a dog.

Example 25

A surface treatment was performed by plasma polymerization following the same procedures as in Example 23 except that a gasket body was prepared by adding 30 parts by weight of carbon black HAF, 1.5 parts by weight of sulfur, 3.0 parts by weight of zinc white, 0.5 parts by weight of stearic acid, and proper amounts of a curing accelerator, a curing assistant agent, and an antioxidant to Solprene 1204 (tradename) available from Nihon Elastomer K.K., curing and press-molding the resultant mixture at 141°C for 30 minutes, and punching the resultant molded body. A measurement and a test were conducted following the same procedures as in Example 23.

The same results as in measurement of the film thickness, the checkerboard test, and measurement of the dynamic friction coefficient as in Example 23 were obtained. No problems occurred in syringe assembly, gamma-ray sterilization, and venous injection to a dog.

Comparative Example 9

No plasma was produced when a voltage was applied following the same conditions as in Example 23 and using the same apparatus and gasket material as in Example 23, except that a monomer was was not supplied. No change on the surface of the gasket. Syringe assembly was impossible without a lubricant.

Comparative Example 10

A plasma was produced in a vacuum of 0.08 Torr following the same procedures as in Comparative Example 9. However, no change occurred on the gasket surface in the absence of a monomer gas. Syringe assembly was impossible without a lubricant.

Example 26

A cured SBR gasket material as in Example 25 was degreased with isopropanol, and polytetrafluoroethylene enamel Polyfron Toughcoat Enamel TC-7400 available from DAIKIN INDUSTRIES, LTD. was coated to a peak portion shown in Fig. 2 and was preliminarily dried at 80°C for 30 minutes. The dried film was sintered at 180°C for 30 minutes. A measurement and a test of a disposable syringe using the resultant gasket was performed following the same procedures as in Example 23. The gasket was cut and a cut surface was observed with a scanning electron microscope. The film had a thickness of 18 to 25 $\mu$m. An adhesion strength based on a checkerboard test was 100/100. A dynamic friction coefficient (with respect to steel) was 0.1. A disposable syringe was easily assembled without a lubricant by using this gasket and a barrel and a plunger which latter two were obtained as in Example 3. No problems occurred in gamma-ray sterilization and venous injection to a dog.

Comparative Example 11

A barrel, a plunger, and a gasket material as in Example 26 were used to assemble a disposable syringe without coating polytetrafluoroethylene to the gasket material. In this case, assembly was impossible without a lubricant. A dynamic friction coefficient was 3.0.

Example 27

A 5-m$\ell$ syringe gasket having a peak diameter of 13.2 mm was injection-molded using an SEBS elastomer AR-804 available from Aron Kasei K.K. 15% by weight of a polyvinylidene fluoride resin KYNAR #301F available from Pennwalt Corp. were dissolved in a 4 : 1 (weight ratio) solvent mixture of acetone and dimethylformamide to prepare a coating solution. The gasket was dipped in the resultant coating solution. The gasket was then dried at room temperature for half a day and at 60°C for an hour, thereby forming a polyvinylidene fluoride film on the surface of the gasket. A plunger obtained by polypropylene MG-3D available from Mitsubishi Petrochemical Co., Ltd. was engaged with the gasket with the polyvinylidene fluoride film. The resultant structure was inserted in a 5-m$\ell$ syringe barrel having an inner diameter of 13.0 mm, thereby obtaining a disposable syringe. The disposable syringe could be assembled without using lubricating silicone oil. A sliding resistance value was 0.25 kgf at a pushing speed of 200 mm/sec. The gasket with the polyvinylidene fluoride film was cut, and the thickness of a film on the cut portion was measured following the same procedures as in Example 23. The thickness was 30 to 25 $\mu$m. Venous injection to a dog was performed using this disposable syringe, following the same procedures as in Example 23. The disposable syringe could be properly used, and no problems occurred. No peeling of the film from the gasket was found.

Example 28

A disposable syringe was assembled and measurements thereof were performed, following the same procedures as in Example 27 except that 10% by weight of a polyvinylidene fluoride resin KYNAR #7201 available from Pennwalt Corp. were dissolved in acetone. A sliding resistance value was 0.23 kgf, and a film thickness was 20 to 25 $\mu$m. Normal use was allowed in venous injection to a dog. No peeling of the film was found.

Example 29

A disposable syringe was assembled and measurements thereof were performed, following the same procedures as in Example 27 except that 8% by weight of a polyvinylidene fluoride resin KYNAR #7201 available from Pennwalt Corp were dissolved in a 1 : 1 (weight ratio) solvent mixture of methyl ethyl ketone and acetone. A sliding resistance value was 0.22 kgf, and a film thickness was 18 to 24 $\mu$m. Normal use was allowed in venous injection to a dog as in Example 23. No peeling of the film was found.

Example 30

A disposable syringe was assembled, and measurements and venous injection to a dog were performed following the same procedures as in Example 27 except that an EP elastomer Milastomer 5510 available from Mitsui Petrochemical Industries, Ltd. The same results as in Example 27 were obtained.

Example 31

A disposable syringe was assembled following the same procedures as in Example 28 except that 35 parts by weight of carbon black HAF, 2.3 parts by weight of sulfur, 5 parts by weight of zinc white, 2 parts by weight of stearic acid, and proper amounts of a curing accelerator, a curing assistant agent, and an were added to 100 parts by weight of isoprene rubber JSR-IR-2200 available from Nihon Gosei Gomu K.K. and the resultant mixture was cured and press-molded at 135°C for 30 minutes. Measurements using this disposable syringe were performed following the same procedures as in Example 27. A sliding resistance value was 0.25 kgf, and a film thickness was 20 to 25 $\mu$m. Normal use was allowed in venous injection to a dog. No peeling of the film was found.

Comparative Example 12

It was attempted to assemble a disposable syringe following the same procedures as in Example 27 except that a 4 : 1 (weight ratio) solvent mixture of acetone and dimethylamide was used in place of a coating solution. However, the resultant gasket could not be inserted in a barrel.

Comparative Example 13

It was attempted to dissolve 5% by weight of a polyvinylidene fluoride resin as in Example 27 in ethyl alcohol but the attempt failed. The resultant solution was used as a coating solution to coat a gasket as in Example 27. However, no film was formed on the surface of the gasket and only a granular material was adhered to the surface.

Example 32

100-$\mu$m thick polyvinylidene fluoride resin (KYNAR #1120 available from Pennwalt Corp) sheet 20 was heated to 180°C and was placed in female mold 21 shown in Figs. 7A, 7B and 7C. Male mold 22 was then inserted in female mold 21, and the mold assembly was deaerated through gas vent port 23 and a vacuum state was maintained in the mold assembly, thereby obtaining lubricating thin film 7 for a gasket. Unnecessary burs were removed from the molded body, and the resultant molded body was inserted in an injection molding mold. An SEBS elastomer AR-804 available from Aron Kasei K.K. was molded by insert injection to prepare a gasket having a peak diameter of 13.2 mm. A disposable syringe was assembled following the same procedures as in Example 27 except that the above gasket was used. As a result, a film on the gasket had a sliding resistance value of 0.3 kgf and a thickness of 30 $\mu$m. Venous injection to a dog as in Example 27 was performed, and normal use of the syringe was allowed. No peeling of the film was found.

Example 33

A disposable syringe was assembled following the same procedures as in Example 32 except that a polyvinylidene fluoride resin KYNAR #730 available from Pennwalt Corp was used. A film on the gasket had a sliding resistance value of 0.28 kgf and a thickness of 30 $\mu$m. Venous injection to a dog was performed, and normal use of the syringe was allowed. No peeling of the film was found.

Example 34

A disposable syringe was assembled and its measurements were performed following the same procedures as in Example 32 except that an EP elastomer Milastomer 5510 available from Mitsui Petrochemical Industries, Ltd. was used in place of the SEBS elastomer. A film had a sliding resistance value of 0.30 kgf and a thickness of 30 $\mu$m. Normal use was allowed in venous injection to a dog. No peeling of the film was found.

Comparative Example 14

It was attempted to assemble a disposable syringe following the same procedures as in Example 32 except that a 500-μm thick sheet of a polyvinylidene fluoride resin sheet as in Example 32 was used. However, a gasket could not be inserted in the barrel.

Comparative Example 15

It was attempted to mold a gasket following the same procedures as in Example 32 except that a 20-μm thick polyvinylidene fluoride resin sheet as in Example 32 was used. However, the front end of a gasket was torn, and a perfect gasket was not molded in vacuum molding.

Example 35

A syringe was prepared using two-color injection molding shown in Figs. 4 and 5. 20 phn of a polytetrafluoroethylene powder (M-12, available from DAIKIN INDUSTRIES, LTD.) were mixed with high-density polyethylene JX20 available from Mitsubishi Petrochemical Co., Ltd. and the resultant mixture was used as a resin having a low dynamic friction coefficient. An ethylene-propylene copolymer thermoplastic elastomer Milastomer 5510 available from Mitsui Petrochemical Industries, Ltd. (hardness JIS-A: 57, compression set (70°C, 22 hours): 41%) was used as a thermoplastic elastomer. The diameter of a gasket was 13.2 mm. A syringe barrel and a plunger were formed using polypropylene MG35 available from Mitsubishi Petrochemical Co., Ltd. and were combined with a gasket to prepare a syringe. The barrel of the syringe had an inner diameter of 13.0 mm. The resultant gasket and barrel could be assembled with the plunger without using polydimethyl silicone SH200 (3,000 cs) available from Toray Silicone K.K.. After 2.0-Hrad gamma-ray sterilization of the syringe, a 22G cannula was attached to the front end of the syringe. A drug liquid was drawn into the syringe and injected in a muscle of a dog, but no problem occurred.

Comparative Example 16

A syringe was manufactured following the same procedures as in Example 35 except that only a thermoplastic elastomer as in Example 35 was used to form a gasket. Although it was attempted to insert the resultant gasket into a barrel, it was impossible without using silicone oil.

Example 36

Molds were heated to 200°C, a polyvinylidene fluoride powder 460 available from Mitsubishi Petrochemical Co., Ltd. and high-density polyethylene (powder type) as in Example 35 were mixed in a 1 : 1 ratio (weight ratio), and the resultant mixture was applied to the molds. A thermoplastic elastomer as in Example 35 was injection-molded to prepare a gasket. The thickness of the resin layer having a low dynamic friction coefficient was about 100 μm. The gasket was used to assemble a disposable syringe as in Example 35. No problems occurred.

Example 37

A polyvinylidene fluoride film (100 μm) having increased adhesion strength was prepared by treating a thermoplastic elastomer contact surface with an alkali. The film was compression-molded by molds and adhered thereto, and then an aerosol type epoxy resin was applied to the film. A thermoplastic elastomer was injection-molded following the same procedures as in Example 35, thereby preparing a gasket. A disposable syringe was assembled following the same procedures as in Example 35, and no problems occurred.

Comparative Example 17

A gasket was manufactured following the same procedures as in Example 37 except that a polypropylene film (100 μm) was used in place of the polyvinylidene fluoride film in Example 37. The polypropylene film had a dynamic friction coefficient of 0.9, and the polyvinylidene fluoride film had a dynamic friction coefficient of 0.22. It was attempted to assemble the gasket with the barrel, but syringe assembly was impossible without silicone oil.

Industrial Applicability

According to a disposable syringe according to the present invention as described above, good sliding properties of the plunger can be obtained without using a lubricant, and dissolution of a lubricant in the syringe content can be prevented. The present invention provides a gasket suitable for use in the disposable syringe and a method of manufacturing the same.

**Claims**

1. A medical instrument comprising :
   a cylindrical member and a sliding member which is adapted to slide along the inner wall of the cylindrical member, wherein the sliding member comprises a thermoplastic elastomer layer and a fluoroplastic resin layer of low dynamic friction coefficient covering a portion of the elastomer which contacts with the inner wall of the cylindrical member,
   characterized in that said fluoroplastic resin layer of low dynamic friction coefficient has a thickness of 5 $\mu$m or less which is formed through a low-temperature plasma polymerization.

2. A medical instrument according to claim 1, wherein the dynamic friction coefficient of the fluoroplastic resin layer is 0.5 or less.

3. A medical instrument according to claim 1, wherein the thermoplastic elastomer is at least one copolymer selected from the group consisting of a styrene-butadiene-styrene-based block copolymer, a styrene-ethylene-styrene-based block copolymer, an ethylene-propylene-based copolymer, a polyester-based copolymer and polyurethane-based copolymer.

4. A medical instrument according to claim 1, wherein a compression set (at 70°C for 22 hours) of the thermoplastic resin according to JIS K6301 is not more than 60%.

5. A medical instrument according to claim 1, wherein a hardness value of the thermoplastic elastomer according to JIS K6301 falls within a range of 35° to 85° in JIS-A.

6. A medical instrument according to claim 1, wherein the fluoroplastic resin layer of low dynamic friction coefficient is selected from the group consisting of polytetrafluoroethylene (PTFE), polyvinylidene fluoride (PVDF), polytetrafluoroethylene containing a perchloroalkoxy side chain (PFA), polychlorotrifluoroethylene, a tetrafluoro-ethylene-hexafluoropropylene copolymer (FEP), a polyvinylethylene fluoride-ethylene tetrafluoride copolymer, high-density polyethylene (HDPE), low-density polyethylene having a very high molecular weight, and a material containing at least one thereof.

7. A medical instrument according to claim 1, wherein the cylindrical member is a barrel of a syringe, and the sliding member is a gasket of the syringe.

8. A medical instrument according to claim 1, wherein said fluoroplastic resin layer has a thickness of 0.3 to 0.5 $\mu$m.

9. A method of manufacturing a medical instrument comprising a cylindrical member and a sliding member which is adapted to slide along an inner wall of the cylindrical member, characterised by the steps of :
   placing a base body of the sliding member which is made of an elastic material and has a predetermined shape, in a monomer gas atmosphere ; generating a plasma in the atmosphere to perform low-temperature plasma polymerization of the monomer, thereby forming a fluoroplastic lubricating film having a thickness of 5 $\mu$m or less on a surface of the base body of the sliding member ; and inserting the sliding member into the cylindrical member.

10. A method according to claim 9, wherein the monomer is a fluorine-containing monomer.

11. A method according to claim 9, wherein a low temperature plasma polymerization pressure in the reaction system falls within a range of $10^{-3}$ to 10 Torr.

EP 0 375 778 B1

**Patentansprüche**

1. Medizinisches Instrument, umfassend:

ein zylindrisches Glied und ein Gleitelement, das zum Gleiten entlang der Innenwand des zylindrischen Gliedes ausgelegt ist, wobei das Gleitelement eine thermoplastische Elastomerschicht und eine einen Teil des Elastomeren, der mit der Innenwand des zylindrischen Gliedes in Berührung steht, bedeckende fluorplastische Harzschicht eines niedrigen dynamischen Reibungskoeffizienten aufweist,

dadurch gekennzeichnet, daß die durch eine Niedrigtemperatur-Plasmapolymerisation gebildete fluorplastische Harzschicht eines niedrigen dynamischen Reibungskoeffizienten eine Dicke von 5 $\mu$m oder weniger aufweist.

2. Medizinisches Instrument nach Anspruch 1, wobei der dynamische Reibungskoeffizient der fluoroplastischen Herzschicht 0,5 oder weniger beträgt.

3. Medizinisches Instrument nach Anspruch 1, wobei das thermoplastische Elastomer aus mindestens einem Mischpolymerisat aus der Gruppe Blockmischpolymerisat auf Styrol/Butadien/Styrol-Basis, Blockmischpolymerisat auf Styrol/Ethylen/Styrol-Basis, Mischpolymerisat auf Ethylen/Propylen-Basis, Mischpolymerisat auf Polyester-Basis und Mischpolymerisat auf Polyurethan-Basis ausgewählt ist.

4. Medizinisches Instrument nach Anspruch 1, wobei die bleibende Verformung (22 h lang bei 70°C) des thermoplastischen Harzes gemäß der japanischen Industrienorm JIS K6301 nicht mehr als 60% beträgt.

5. Medizinisches Instrument nach Anspruch 1, wobei der Härtewert des thermoplastischen Elastomeren gemäß der japanischen Industrienorm JIS K6301 im Bereich von 35° bis 85° nach JIS-A liegt.

6. Medizinisches Instrument nach Anspruch 1, wobei die fluorplastische Harzschicht eines niedrigen dynamischen Reibungskoeffizienten aus der Gruppe Polytetrafluorethylen (PTFE), Polyvinylidenfluorid (PVDF), Polytetrafluorethylen mit einer Perchloralkoxyseitenkette (PFA), Polychlortrifluorethylen, ein Tetrafluorethylen/Hexafluorpropylen-Mischpolymerisat (FEP), ein Polyvinylethylenfluorid/Ethylentetrafluorid-Mischpolymerisat, hochdichtes Polyethylen (HDPE), niedrig dichtes Polyethylen mit einem sehr hohem Molekulargewicht und ein mindestens einen Bestandteil davon enthaltendes Material, ausgewählt ist.

7. Medizinisches Instrument nach Anspruch 1, wobei das zylindrische Glied aus einem Spritzenzylinder und das Gleitelement aus einem Dichtring der Spritze bestehen.

8. Medizinisches Instrument nach Anspruch 1, wobei die fluorplastische Harzschicht eine Dicke von 0,3 bis 0,5 $\mu$m aufweist.

9. Verfahren zur Herstellung eines medizinischen Instrumentes mit einem zylindrischen Glied und einem Gleitelement, das zum Gleiten entlang einer Innenwand des zylindrischen Elements ausgelegt ist, gekennzeichnet durch die folgenden Schritte:

Anordnen eines Grundkörpers des Gleitelements aus einem elastischen Material einer vorbestimmten Form in einer Monomerengasatmosphäre,

Erzeugen eines Plasmas in der Atmosphäre zur Durchführung einer Niedrigtemperatur-Plasmapolymerisation des Monomeren um einen fluorplastischen Gleitfilm einer Dicke von 5 $\mu$m oder weniger auf einer Oberfläche des Grundkörpers des Gleitelements auszubilden, und

Einführen des Gleitelements in das zylindrische Glied.

10. Verfahren nach Anspruch 9, wobei das Monomere aus einem fluorhaltigen Monomeren besteht.

11. Verfahren nach Anspruch 9, wobei der Niedrigtemperatur-Plasmapolymerisationsdruck im Reaktionssystem im Bereich von $10^{-3}$ bis 10 Torr liegt.

16

**EP 0 375 778 B1**

**Revendications**

1. Instrument médical comprenant :
   un élément cylindrique et un élément glissant qui est adapté pour glisser le long de la paroi interne de l'élément cylindrique, dans lequel l'élément glissant comprend une couche d'élastomère thermoplastique et une couche de résine plastique fluorée de faible coefficient de frottement dynamique recouvrant une partie de l'élastomère qui est en contact avec la paroi interne de l'élément cylindrique, caractérisé en ce que ladite couche de résine plastique fluorée de faible coefficient de frottement dynamique a une épaisseur de 5 $\mu$m ou moins qui est formée par polymérisation en phase plasma à basse température.

2. Instrument médical selon la revendication 1, dans lequel le coefficient de frottement dynamique de la couche de résine plastique fluorée est de 0,5 ou moins.

3. Instrument médical selon la revendication 1, dans lequel l'élastomère thermoplastique est au moins un copolymère choisi parmi les copolymères séquencés à base de styrène-butadiène-styrène, les copolymères séquencés à base de styrène-éthylène-styrène, les copolymères à base d'éthylène-propylène, les copolymères à base de polyesters et les copolymères à base de polyuréthannes.

4. Instrument médical selon la revendication 1, dans lequel la déformation permanente (à 70°C pendant 22 h) de la résine thermoplastique selon la norme JIS K6301 n'est pas supérieure à 60 %.

5. Instrument médical selon la revendication 1, dans lequel la dureté de l'élastomère thermoplastique selon la norme JIS K6301 est dans la gamme de 35 à 85° en JIS-A.

6. Instrument médical selon la revendication 1, dans lequel la couche de résine plastique fluorée de faible coefficient de frottement dynamique est choisie parmi le polytétrafluoroéthylène (PTFE), le fluorure de polyvinylidène (PVDF), le polytétrafluoroéthylène contenant une chaîne latérale perchloroalcoxy (PFA), le polychlorotrifluoroéthylène, les copolymères tétrafluoroéthylène-hexafluoropropylène (FEP), les copolymères fluorure de polyvinyléthylène-tétrafluorure d'éthylène, le polyéthylène haute densité (HDPE), le polyéthylène basse densité de très haut poids moléculaire et une substance contenant l'un au moins de ces composés.

7. Instrument médical selon la revendication 1, dans lequel l'élément cylindrique est un cylindre de seringue et l'élément glissant est un joint de la seringue.

8. Instrument médical selon la revendication 1, dans lequel ladite couche de résine plastique fluorée a une épaisseur de 0,3 à 0,5 $\mu$m.

9. Procédé de fabrication d'un instrument médical comprenant un élément cylindrique et un élément glissant qui est adapté pour glisser le long de la paroi interne de l'élément cylindrique, caractérisé par les étapes suivantes : on place un corps de base de l'élément glissant qui est fait d'une matière élastique et qui a une forme prédéterminée dans une atmosphère de gaz monomère ; on produit un plasma dans l'atmosphère pour effectuer une polymérisation du monomère dans le plasma à basse température, en formant ainsi un film plastique fluoré lubrifiant ayant une épaisseur de 5 $\mu$m ou moins sur la surface du corps de base de l'élément glissant ; et on introduit l'élément glissant dans l'élément cylindrique.

10. Procédé selon la revendication 9, dans lequel le monomère est un monomère fluoré.

11. Procédé selon la revendication 9, dans lequel la pression de polymérisation dans le plasma à basse température dans le système de réaction est dans la gamme de $10^{-3}$ à 10 Torr.

17

Fig.1.

Fig.2.

Fig.3.

Fig.4.

Fig.5.

Fig. 6.

(A)

(B)

(C)

Fig.7.

EP 0 375 778 B1